Europäisches Patentamt

**European Patent Office**

Office européen des brevets

(19)

(11) Publication number: **0 282 316**
**A2**

(12)

# EUROPEAN PATENT APPLICATION

(21) Application number: **88302115.6**

(22) Date of filing: **10.03.88**

(51) Int. Cl.⁴: **A 61 L 25/00**

(30) Priority: **11.03.87 US 24268   11.03.87 US 24265**

(43) Date of publication of application:
**14.09.88 Bulletin 88/37**

(84) Designated Contracting States:
**CH DE FR GB IT LI**

(71) Applicant: **MEDI-TECH INTERNATIONAL CORPORATION**
**26 Court Street**
**Brooklyn New York 11242 (US)**

(72) Inventor: **Cornell, John**
**1306 Birmingham Road**
**West Chester Pennsylvania 19382 (US)**

(74) Representative: **Duncan, Angus Henry**
**Barker, Brettell & Duncan 138 Hagley Road**
**Edgbaston Birmingham, B16 9PW (GB)**

(54) **Wound dressings in sheet or gelled paste form.**

(57) A gel composition in the form either of a transparent sheet or paste is provided for dressing skin wounds. When the dressing is in the form of a sheet, the composition comprises dextrin and a water-swellable, cross-linked polymer selected from polyacrylamide or polymethacrylamide. The ratio of dextrin to polymer ranges from about 2:1 to 1:1. When the dressing is in the form of a paste the composition comprises a non-cross-linked nonionic polyacrylamide or polymethacrylamide in combination with a cross-linked salt of polyacrylic or polymethacrylic acid and water. The ratio of nonionic to cross-linked polymer is from about 1:2.5 to 1:15; while the ratio of water to combined nonionic and cross-linked polymer is from about 10:1 to 1:3. Glycerine can be a further component of the paste and, when present, will be found in a ratio of water to glycerin ranging from 5:1 to 1:0.7.

EP 0 282 316 A2

Bundesdruckerei Berlin

**Description**

## WOUND DRESSINGS IN SHEET OR GELLED PASTE FORM

Field of the Invention
The invention concerns novel skin dressings which aid in the healing of wounds.

Background of the Invention
Burns, ulcerations, severe abrasions, skin transplants and similar poorly healing wounds affecting relatively large areas of skin are particularly vulnerable to infection. Artificial skins have been developed as bandages to temporarily protectively cover these wounds. These bandages must promote biofixation, control bacterial growth, supply moisture and prevent evaporation.

U.S. Patent 3,849,238 (Gould et al.) discloses artificial skin comprising a water-containing hydrophilic polymer sponge layer and a thinner hydrophobic polymer layer. Illustrative of the hydrophilic polymers are hydroxyalkyl acrylates or methacrylates, acrylamides, and derivatives thereof. The hydrophobic component may be alkoxyalkyl acrylates or methacrylates, vinlyacetate polymers, elastomeric silicone or polymerized olefins such as polyisoprene, polybutadiene or polyethylene.

U.S. Patent 4,248,685 (Beede et al.) reports aqueous hydrocolloidal dispersions of random interpolymers having bacteriostatic properties. These interpolymers can initially be prepared as gels. Gelled material can then be cast into a self-supporting, transparent, conformable film wound dressing. The interpolymers are derived from the polymerization of a monomer mixture comprising 10-90% alpha, beta-olefinically unsaturated carboxylic acid esters with 90-10% alpha, beta-olefinically unsaturated amides capable of being dispersed in water. A difunctional monomer such as $N,N^1$-methylene bisacrylamide must also be present to cross-link the polymer mixture.

U.S. Patent 4,554,317 (Behar et al.) describes a synthetic hydrophilic membrane for use as a wound covering. The membrane is prepared by graft polymerization of a hydrophilic monomer with a polyurethane substrate. Included among the hydrophilic monomers are acrylamides, hydroxyalkyl acrylates and hydroxyalkyl methacrylates. Gamma radiation and x-rays are suggested as suitable for initiating the graft polymerization.

Wound dressings described in the foregoing art are frequently opaque. Visual observation of the healing process is therethrough prevented. Infection or other complications cannot be detected with opaque coverings. Transparent wound dressings would therefore be highly desirable.

Transparent wound dressings are known. A particularly useful form is the gel type which can be cast as a sheet or strip. This type is commercially available, for instance, from Geistlich-Pharma Inc., Atlanta, Georgia, under the trademark of GELIPERM. Sheets of strips have the advantage of easy usage, removability without disturbing healing, and promoting improved healing without excess granuloma formation.

U.S. Patent 4,556,056 (Fischer et al.) discloses transparent gels in sheet of strip form for use as fluid bandages. These sheets are produced by dissolving a monomer and a gellable polysaccharide in water and therein initiating free-radical polymerization of the monomer. While the compositions of this patent substantially improve over the cited art, problems do remain. In particular, the sheets of Fischer et al. as embodied in the GELIPERM product have clinically exhibited too rapid an uptake of moisture. Moreover, the preferred polysaccharides described therein tend to gel too severely on wetting causing greater difficulty in handling the components as they are cast into sheets. Control of sheet quality and rate of production also thereby suffer.

As noted from the art cited above, most compositions are intended to be either powder compositions, membranes or cast sheet type products. These formulations do not take into consideration the special problems of gelled pastes deliverable from tubes. These pastes must be sufficiently gelled to avoid being of soft, limp, flowable consistency. Pastes which run will not adequately adhere to a wound. On the other hand, there is the danger of over gellation. Pastes which are too thick are difficult to extrude from the containers. There is also the aesthetic problem when dealing with too loose or too thick paste formulations.

Water-absorption rates have also not readily been controllable with the gelled paste compositions of the prior art. It is important that the rate of fluid uptake be neither too rapid nor too slow. A modest controlled rate of moisture absorption must be exhibited by such products.

Consequently, it is an object of this invention to provide a wound dressing in either transparent sheet or paste form which overcome the problems mentioned with such products.

Another subject of this invention is to provide a formulation that when in sheet form is easily handleable for casting.

Another object of this invention is to provide a dressing in paste form which is neither too limp nor too thick for dispensing through tubes and which readily adheres to the wound area.

A still further object of this invention is to provide a dressing in paste form which has a moderate, controlled rate of fluid absorption.

Summary of the Invention
These and other objects have been met by a gel composition for dressing skin wounds in the form of a transparent sheet or paste comprising:
    (a) a non-cross-linked nonionic structuring agent;

and

(b) a water-swellable cross-linked organic polymer.

More particularly, when the gel composition is in the form of a transparent sheet, the composition comprises:

(i) a dextrin; and

(ii) a water-swellable cross-linked polymer selected from polyacrylamide or polymethacrylamide, wherein the ratio of dextrin to polymer ranges from about 2:1 to 1:1.

The gel composition of this invention may also be in the form of an extrudible gelled paste comprising: (i) a non-cross-linked polymer selected from a polyacrylamide or polymethacrylamide;

(ii) a cross-linked salt of a polycarboxylic acid selected from polyacrylic or polymethacrylic acids; and

(iii) water;

wherein the ratio of (i) to (ii) ranges from about 1:2.5 to about 1:15, and the ratio of (iii) to the sum of (i) and (ii) ranges from about 10:1 to 1:3.


Detailed Description of the Invention

Wound dressings of the present invention may either be in sheet or gelled paste form. When in sheet form, it has been discovered that the saccharide dextrin is unexpectedly effective as the nonionic structuring agent for controlled swelling of the wound dressing when combined with certain organic polymers. Dextrin is a non-cross-linked carbohydrate intermediate between starch and the sugars. The material is produced from starch through hydrolysis by dilute acids, distase or dry heat. Other common names for dextrin are British gum, starch gum, amylin and gummeline; the general structure is $(C_6H_{10}O_5)_x$ wherein x may be a value from 6 to 7. The saccharide may be obtained commercially from the American Maize-Products Company, a preferred grade being known as Dextrin 1104.

Unlike gelling agents such as agar-agar, dextrin does not gel when wetted. Immediate gelling is undesirable. Delayed gellation permits sufficient time for liquid absorption by the entire composition and aids the swelling agent to produce controlled fluid sorption by the polymer. Thus, there is greater tolerance in preparation of the dressing. Lumping during fluid absorption is thereby avoided. A beneficial aspect of dextrin containing wound dressings as herein described, is the superior water absorption profile (i.e. controlled wetting). Similarly formulated gels employing sorbitol or gelatin were found to have much slower rates of moisture absorption than the dextrin compositions. Mono and disaccharides have little structure in the fluid swollen gel, while starch or cellulosics do not aid in fluid absorption control.

A second important component of the dressing of this invention is a water-swellable hydrophilic polymer. Cross-linked, water swellable polyacrylamides or polymethacrylamides have been determined to be the most suitable hydrophilic polymers for the sheet product form. Generally, these polymers may be obtained by free-radical polymerization of acrylamide (or methacrylamide) using an appropriate radical initiator catalyst. Appropriate catalysts include sodium monopersulfate, sodium percarbonate, and other water soluble hydrogen peroxide generators. Under certain circumstances there may alternatively be employed organic nitrogen type radical initiators such as azobisisobutyronitrile. Gamma radiation and x-rays may also be utilized to initiate polymerization in a water phase; irradiation will then further induce cross-linking.

Peroxides or nitrogen type initiators themselves may require a promotor or activator to begin generation of free-radicals at ambient conditions. Amines are highly effective promotors of peroxide decomposition. A preferred amine for use with persulfate is tetramethylethylene diamine. Initiation or promotion of nitrogen type initiators is best done with heat or ultraviolet radiation.

Besides the acrylamide or methacrylamide monomer, there must also be present a cross-linking agent. Particularly preferred for cross-linking is methylenebisacrylamide. Of course, other difunctional monomers may also be useful including divinylbenzene or ethylenedimethacrylate.

Another important, in fact critical, parameter in forming the dressing is the relative amount of nonionic structuring or swelling agent to polymer. The nonionic structuring or swelling agent polymer ratio must lie between 2:1 to 1:1. Preferably, the ratio ranges between 2:1 and greater than 1:1, ideally about 1.5:1 to 1.1:1.

In concentration terms, the weight of cross-linked polyacrylamide or polymethacrylamide will comprise from about 1% to 20% by weight of the final dressing. Preferably, the polymer concentration will range from about 5% to 15%, optimally about 10%. Dextrin will similarly be present from about 1% to 20% by weight of the final dressing; preferably 5% to 15%, optimally about 10%.

Other components of the composition include water found in amounts from about 50% to 90%; preferably, from 65% to 80%.

Wound dressings of the present invention may also be a granulated gel in the form of a paste. The paste is an inert hydrogel composed of a sterile solution of a polymeric structurant and incorporating a special moisture absorbent and a cross-linked polymer. The granulated gel provides a moist environment to aid in the healing process. Granules of the paste form a gel on the wound; these granules will then continuously soften in the presence of moisture. They have the ability to absorb wound exudate, thereby acting as a wound cleansing agent. Exudate diffuses into the gel which assists in the removal of contaminants from the wound. Gentle swabbing of the wound with a saline solution easily removes the gel in preparation for a fresh application of gel.

The compositions of the present invention are particularly effective in encouraging granulation and epithelization over chronic ulcers, especially ulcers cruris and decubitis ulcers.

The polymeric structurant of the paste product utilizes a non-cross-linked essentially nonionic polyacrylamide or polyethacrylamide homopolymer. These polymers are commercially available from the American Cyanamide Company. Especially preferred is Gelamide 250 which is a homopolymer of acrylamide having a molecular weight of approximately five to six million. Low molecular weight polymers have been found to be unsuitable as structurants. Thus, it is important that the polyacrylamide or polymethacrylamide have a molecular weight within the range of one to ten million; preferably the range should be from about five to eight million. When the molecular weight is too low the gelled paste becomes soft, limp, extremely flexible, and separates into small pieces; the paste flows as a lumpy fluid.

The amount of polyacrylamide or polymethacrylamide will generally range from about 0.1 to about 20% by weight of the composition;preferably from about 0.5 to 10%; optimally from about 0.8 to about 2%.

The moisture absorbing water-swellable organic polymer which is the second crucial element of the compositions is, in the case of the paste product, a salt of a polyacrylic or polymethacrylic acid which must be highly cross-linked. Polyacrylic or polymethacrylic acids which are lightly or non-cross-linked were found to dissolve in water very rapidly which resulted in a weak gel. A swellable bead form of polyacrylic acid is preferred for purposes of the present invention.

Appropriate counterions for polyacrylic or polymethacrylic acid are sodium, potassium, lithium ,ammonium, substituted ammonium, and cation mixtures thereof. These polymeric water absorbent salts may be present in amounts from about 1% to 20%, preferably from about 3% to about 15%; optimally from about 6% to about 10% by weight of the composition.

Sterile water is the major constituent of the gelled paste. Appropriate water concentrations will range from about 60% to about 95%; preferably from about 70% to about 85%; optimally from about 70% to about 75% by weight of the total composition.

Usually the compositions will also contain a moisture regulating, viscosity controlling agent in the form of a fluid polyol. Not all types of polyols are, however, suitable for the present wound dressing. Glycerine is especially suitable as a moisture control, viscosity improving agent. This polyol retards the water absorption rate so as to render the initial formulation more stable and of increased viscosity. As a result, the dressing components assemble into a flowable paste of suitable body. Other polyols such as polyethylene glycols are normally not employed as the polyol because of limited biological compatability with the human body.

In another aspect of this invention, it has been discovered that the relative amounts of the major components of the paste product must be present within critical ratios. Thus, the ratio of polyacrylamide (or polymethacrylamide) to polyacrylic (or polymethacrylic) acid salt will range from about 1:2.5 to 1:15; preferably from about 1:4 to 1:10; optimally about 1:8.

There is also a critical ratio between the amount of water and the combined amounts of polyacrylamide (or polymethacrylamide) and polyacrylic (or polymethacrylic) acid salt. Broadly the critical ratio ranges from 10:1 to 1:3. There is also a critical ratio between the amount of water and glycerine. For these two components the critical ratio varies from about 5:1 to 1:0.7, respectively; preferably about 2:1 to 1:1.

Compositions containing glycerine will incorporate this component in amounts from about 2% to 50% by weight; preferably from about 10% to 30%; optimally from about 15% to 25%.

Earlier it was mentioned that the gelled paste must have a substantially rigid character; the paste must not be easily flowable. A suitable viscosity for the water solution will range from about 10,000 to 100,000 centipoise when measured at the level of 2.5% in water by a Brookfield viscometer (spindle 3, 6 rpm) at 25°C.

Application of the aforedescribed compositions to a wound is preceded by wound preparation. First there is a debridement of necrotic tissue, a cleansing and, where necessary, application of a topical antiseptic. Thereupon, the gelled paste is applied in a layer 0.05 cm thick (minimum) of until the wound cavity is filled. If desired, the granulant can be loosely covered with a dry dressing. Sufficient room must, however, be left for expansion as exudate and contaminants are absorbed.

Dressing changes should be carried out once or twice daily. Where there is more heavily contaminated or exuding wounds, more frequent changes may be necessary. When the overlaying dressing is removed, some of the gelled paste will cling thereto. Any residue is best removed by gentle irrigation of the wound with a saline. Gentle swabbing of the wound with saline will remove any granulate residue. This prepares the wound for a fresh application of the gelled paste.

The following examples will more fully illustrate various embodiments of the present invention, all parts and percentages therein being by weight, unless otherwise noted.

Example 1

A typical gelled paste formulation is outlined in the table below.

## Table I

### Gelled Paste Composition

| Component | Parts by Weight |
|---|---|
| Gelamide 250 | 1 |
| Glycerine | 20 |
| Cross-linked Polyacrylic Acid | 8 |
| Sterile Water | 72 |

A Hobart or planetary mixer is used for stirring the components listed in the Table. Gelamide 250 is added slowly to the glycerine while mixing. Water is then added. Initially, room temperature water is used but then the remaining major portion of water should be added at 60°C. The hot water enhances the dissolving of the gelamide. Mixing is then continued until a smooth homogeneous composition is attained. This composition is cooled to room temperature. Polyacrylic acid is then combined with the other components, care being taken to minimize mixing. There results a product which is a flowable paste whose consistency is similar to that of toothpaste. Thereafter, the gelled paste is packaged in laminated toothpaste-type tubes.

Example 2
A typical sheet formulation is outlined in the table below.

## Table II

### Transparent Sheet Formulation

| | Components | Weight (grams) | Percent |
|---|---|---|---|
| Part I: | Acrylamide | 400 | 10 |
| | Dextrin 1104 | 400 | 10 |
| | Methylenebisacrylamide | 0.35 | 0.08 |
| | Tetramethylethylene diamine | 0.4 | 0.1 |
| | Distilled Water | 3,000 | 75 |
| Part II: | Distilled Water | 180 | 5 |
| | Sodium Persulfate | 20 | 0.05 |
| | Total | 4,000 | |

Distilled water, in an amount listed in Part I, is heated to 60°C. Dextrin is slowly added thereinto with mixing until there is complete dissolution. Thereafter, the remaining components including acrylamide, methylenebisacrylamide, and tetramethylethylene diamine. Part I is stable for several days at room temperature. Part II is an aqueous solution of the free-radical initiator, sodium persulfate; this solution is stable for at least several weeks.

When ready to fill trays, Part I is mixed with Part II at 60°C. The preferred blending method is to fill the trays directly fro a motionless mixer. These trays are similar to commercial polyethylene meat trays. Prior to being filled, each tray is coated with a release agent. Silicone or Pam may be used as the release agent.

Each tray is filled with about 100-150 grams and allowed to cure. Curing occurs rapidly (within 3-15 minutes).

Post cure, when desired for removal of residual acrylamide monomer, is usually performed by placing the trays in an oven for 3 hours at 60°C.

<u>Example 3</u>
The following example illustrates the effect on transparent sheet products of other swelling agents on water absorption when combined with polyacrylamide. Sheets were cast in a process similar to that described in Example 2. The compositions are outlined in Table III. Water absorption values may be found in Table IV.

## Table III

### Acrylamide-Swelling Agent Compositions

| Part I: | Components | Composition Number (Weight-Grams) | | |
|---|---|---|---|---|
| | | **1** | **2** | **3** |
| | Acrylamide | 100 | 100 | 100 |
| | Methylenebisacrylamide | 0.17 | 0.17 | 0.17 |
| | Tetramethylethylene diamine | 0.1 | 0.1 | 0.1 |
| | Distilled Water | 750 | 750 | 750 |
| | Dextrin 1104 | 100 | — | — |
| | Sorbitol | — | 100 | — |
| | Gelatin | — | — | 100 |
| Part II: | Distilled Water | 45 | 45 | 45 |
| | Ammonium Persulfate | 5 | 5 | 5 |

## Table IV

### Water Absorption Rates

| Composition No. | Swelling Agent | Water Absorption (%) | Time (min.) |
|---|---|---|---|
| 1 | Dextrin | 65 | 2 |
| | | 100 | 3.5 |
| | | 109 | 5 |
| | | 120 | 10 |
| 2 | Sorbitol | 40 | 10 |
| | | 60 | 20 |
| | | 80 | 30 |
| | | 90 | 40 |
| | | 120 | 120 |
| 3 | Gelatin | 13 | 10 |
| | | 19 | 20 |
| | | 20 | 40 |

From Table IV it is evident that gelatin-polyacrylamide sheets have a very low,and inadequate, rate of water absorption. Only 20% water was taken up after a full 40 minutes. Sorbitolpolyacrylamide sheets exhibited better, but still inadequate, water absorption properties. After 40 minutes, absorption reached 90%. By contrast, the dextrin-polyacrylamide compositions had a water absorption of 100% after 3.5 minutes.

Various modifications and changes may be made with regard to the foregoing detailed description without departing from the spirit and scope of this invention.

**Claims**

1. A gel composition for dressing skin wounds in the form of a transparent sheet or paste comprising:
   (a) a non-cross-linked nonionic structuring agent; and
   (b) a water swellable cross-linked organic polymer.

2. A composition according to Claim 1 which is in the form of a transparent sheet and comprises:
   (i) a non-cross-linked nonionic structuring agent which is dextrin; and
   (ii) a water-swellable, cross-linked organic polymer selected from polyacrylamide or polymethacrylamide, wherein the ratio of dextrin to polymer is from about 2:1 to 1:1.

3. A composition according to Claim 2 wherein the ratio of dextrin to polymer ranges between 1.5:1 to 1.1:1.

4. A composition according to Claim 2 having water present in an amount from about 50% to 90% by weight.

5. A composition according to Claim 1 in the form of a gelled paste which comprises:
   (i) a non-cross-linked nonionic polyacrylamide or polymethacrylamide;
   (ii) a water-swellable cross-linked polymer which is a salt of polyacrylic or polymethacrylic acid; and
   (iii) water:
   wherein the ratio of (i) to (ii) ranging from about 1:2.5 to 1:15 and the ratio of (iii) to the sum of (i) plus (ii) ranging from about 10:1 to 1:3.

6. A composition according to Claim 5 wherein there is further present an amount of glycerin, the ratio of water to glycerin being from about 5:1 to 1:0.7.

7. A composition according to Claim 5 wherein the ratio of water to glycerin ranges from about 2:1 to 1:1 and the ratio of (i) to (ii) ranges from about 1:4 to 1:10.

8. A composition according to Claim 5 having a viscosity ranging from about 10,000 to 100,000 centipoise when measured in water at 2.5% concentration using a Brookfield viscometer at 25°C.

9. A composition according to Claim 5 wherein the polyacrylamide or polymethacrylamide has a molecular weight ranging from about one to ten million.